# EUROPÄISCHE PATENTSCHRIFT

(11) **EP 0 427 055 B1**
(45) Veröffentlichungstag und Bekanntmachung des Hinweises auf die Patenterteilung: **16.02.1994**
(21) Anmeldenummer: 90120527.8
(22) Anmeldetag: 26.10.1990
(51) Int. Cl.: A61L 2/18

(54) **Verfahren und Vorrichtung zur Sterilisation einer medizinischen Baugruppe**
Process and appliance for sterilising a medical assembly group
Procédé et installation pour la stérilisation d'un assemblage médical

(30) Priorität: 04.11.1989 DE 3936785
(43) Veröffentlichungstag der Anmeldung: 15.05.1991
(73) Patentinhaber: Fresenius AG, 61350 Bad Homburg (DE)
(72) Erfinder: Heilmann, Klaus, W-6690 St. Wendel (DE); Mathieu, Bernd, Dr. Dipl.-Chem., W-6683 Spiesen-Elversberg (DE); Rink, Michael, Dr., W-6670 St. Ingbert-Hassel (DE); Weber, Wolfram, W-6683 Spiesen-Elversberg (DE)
(74) Vertreter: Fuchs Mehler Weiss

(56) Entgegenhaltungen:
- EP-A- 0 198 100
- EP-A- 0 370 850
- DE-A- 3 708 733
- DE-A- 3 708 734
- GB-A- 1 575 377

## Beschreibung

Die Erfindung bezieht sich auf ein Verfahren zur Sterilisation einer medizinischen Baugruppe mit mindestens zwei Anschlüssen zur Einschaltung in einen Flüssigkeitsstrom eines medizinischen Gerätes, bei dem die Baugruppe von einem Sterilisiermedium durchströmt wird.

Die Erfindung bezieht sich ferner auf eine Vorrichtung zur Durchführung dieses Verfahrens mit einer Einrichtung zur Erzeugung und/oder Bereitstellung des Sterilisiermediums im System.

Derartige medizinische Baugruppen, wie beispielsweise medizinische Filter, insbesondere Blutbehandlungsfilter, die in den extrakorporalen Kreislauf eingeschaltet werden (z. B. Dialysatoren), müssen im hohen Maße dauerhaft steril sein. Gleiches gilt für Baugruppen, die bei der Applikation steriler Medikamente Anwendung finden.

Es ist in der Praxis bekannt, derartige medizinische Baugruppen in aufeinanderfolgenden Schritten versandfertig zu sterilisieren. Die einzelnen Schritte erfolgen jeweils räumlich bzw. anlagemäßig isoliert voneinander, so daß zwischen den einzelnen Schritten unsterile Zwischenschritte liegen, bei denen die Sterilität verlorengehen kann.

So wird bei einem aus der DE 30 12 110 A1 bekannten Verfahren eine künstliche Niere zunächst mit einer für den menschlichen Körper unschädlichen Flüssigkeit vollständig ausgefüllt. Danach werden die Anschlüsse der Niere verschlossen. Anschließend wird die bereits in einem Verpackungsbehälter untergebrachte künstliche Niere in einen Autoklaven eingebracht und in Dampf unter hoher Temperatur und hohem Druck sterilisiert.

Wesentliche typische Nachteile des bekannten Verfahrenstyps sind:
- Man benötigt teure, dehnbare Spezialverschlüsse bzw. Ausdehnungskammern, um die Volumenvergrößerung bei der Sterilisation im Autoklaven auffangen zu können;
- die Verschlüsse können dennoch reißen;
- es kann nicht jeder Typ von künstlichen Nieren nach dem bekannten Verfahren sterilisiert werden;
- da quasi bei "stehender Flüssigkeit" in der künstlichen Niere sterilisiert wird, sind nicht-keinfreie Nester in toten Winkeln mit Wärmebrücken nicht auszuschließen.

Einen Schritt weiter geht das aus der EP 0 198 100 A 1 bekannt gewordene Verfahren, bei dem ein künstliches menschliches Organ, z. B. eine künstliche Niere, in einem Autoklaven von innen mit auf Steriltemperatur erhitztem Wasser im Durchlaufbetrieb und von außen mit Heißdampf sterilisiert wird. Anschließend wird das Organ durch ein entsprechend kühles steriles Wasser, welches das Organ innen durchfließt, unter Erhalt der Sterilität abgekühlt.

Von diesem bekannten Verfahren mit Sterilisation im Durchlaufbetrieb geht die Erfindung aus.

Wesentlicher Nachteil des bekannten Verfahrens und der zugehörigen Vorrichtung ist, daß bei Entnahme des künstlichen Organs aus dem Autoklaven zwecks Verschließen und Verpacken des Organs die Sterilität verlorengehen kann.

Der Erfindung liegt die Aufgabe zugrunde, das eingangs bezeichnete Verfahren sowie die bezeichnete Vorrichtung so weiterzubilden, daß unsterile Zwischenschritte vermieden werden.

Die Lösung dieser Aufgabe gelingt bei dem Verfahren gemäß der Erfindung durch folgende Verfahrensschritte:
a. Einsetzen der Baugruppe in ein System unter druckdichtem Ankoppeln der beiden Anschlüsse an eine Zu- und Ableitung des Systems unter atmosphärischen Bedingungen,
b. Durchströmen der Baugruppe mit dem Sterilisiermedium aus dem System für einen vorgegebenen Zeitraum,
c. Keimdichtes Verschließen der beiden Anschlüsse im angekoppelten Zustand ohne diese zu öffnen nach Abschluß der Sterilisation,
d. Entnahme der Baugruppe aus dem System als sterile Einheit mit verschlossenen Anschlüssen.

Bei der Vorrichtung gelingt die Lösung gemäß der Erfindung durch
- mindestens eine Atmosphärendruck ausgesetzte Spannstation für die temporäre druckdichte Ankopplung der Baugruppen-Anschlüsse, welche Spannstation einen Zulauf und einen Abfluß aufweist,
- mindestens eine Rohrleitung von der Sterilisiermedium-Einrichtung zum Zulauf der Spannstation,
- mindestens eine Rohrleitung, die am Abfluß der Spannstation angeschlossen ist,
- Verschlußorgane, insbesondere Absperrventile, in den Rohrleitungen,
- zumindest eine Einrichtung zum keimdichten Verschließen der Anschlüsse der Baugruppe im angekoppelten Zustand.

Bei dem Verfahren und der Vorrichtung nach der Erfindung erfolgt somit die Sterilisation "inline", d.h. alle Behandlungsschritte werden im selben System durchgeführt, in dem auch die Behandlungsmittel durchlaufen. Dadurch ist gewährleistet, daß keine insterilen Zwischenschritte auftreten und medizinische Baugruppen den Patienten erreichen, die mit einem Höchstmaß an Sicherheit steril verschlossen sind.

Als Sterilisiermedien kommen die üblichen Mittel in Betracht, z. B. Druckwasser oder Wasserdampf von mindestens 105°C, je nach Verträglichkeit mit der jeweiligen medizinischen Baugruppe. Für Baugruppen, die hydrophob sind, kommt als Sterilisiermedium mit Vorteil eine Peressigsäurelösung in Betracht.

Bei bestimmten medizinischen Baugruppen in Verbindung mit dem anzuwendenden Sterilisiermedium ist ein Spülschritt vor und/oder nach dem Schritt der Sterilisation notwendig. Als Spülflüssigkeit findet vorzugsweise Wasser Verwendung, das bei einer Spülung nach der Sterilisation selbst steril sein muß.

Bei einem geschlossenen System mit Druckwasser oder Heißdampf als Sterilisiermedium kann das Spülwasser aus dem Sterilisiermedium abgeleitet werden, z. B. dient das noch nicht hocherhitzte Wasser als Spülflüssigkeit vor dem Schritt der Sterilisation sowie abgekühltes Druckwasser bzw. kondensierter Wasserdampf als Spülflüssigkeit nach der Sterilisation.

Je nach medizinischer Baugruppe ist es zweckmäßig, diese bei ihrem Verschließen mit Sterilflüssigkeit befüllt zu lassen oder einen Trocknungsschritt mit sterilem Gas, z. B. Luft, die gegebenenfalls auf mindestens 100°C erhitzt wird, zwischenzuschalten.

Je nach vorgegebener medizinischer Baugruppe ergeben sich daher verschiedene Kombinationsmöglichkeiten.

Bei hydrophilen Baugruppen, z. B. einem Cuprophan-Dialysator, wird das Verfahren in der Weise durchgeführt, daß der Spülvorgang mit heißem Wasser durchgeführt wird, daß heißes Wasser unter Druck anschließend auf Steriltemperatur erhitzt und mit diesem Druckwasser danach im Umlauf der Sterilisationsschritt als Heißsterilisation durchgeführt wird, daß anschließend das umlaufende Druckwasser auf Raumtemperatur abgekühlt und danach die medizinische Baugruppe unter Sterilbedingungen verschlossen wird.

Bei einer hydrophoben Baugruppe, z. B. einem Polysulfon-Membranfilter, wird das Verfahren mit Vorteil in der Weise durchgeführt, daß der Spülvorgang mit heißem Wasser durchgeführt wird, daß danach mit Wasserdampf als Heißsterilisiermedium der Sterilisationsschritt durchgeführt wird, und daß die medizinische Baugruppe anschließend unter Sterilbedingungen verschlossen wird. Zweckmäßig wird dabei die Baugruppe nach der Heißsterilisation mit Sterilwasser nachgespült und anschließend getrocknet.

Alternativ zu diesem Verfahren mit Druckwasser kann das Verfahren auch in der Weise durchgeführt werden, daß der Spülvorgang mit heißem Wasser durchgeführt wird, daß danach mit Peressigsäure bei Raumtemperatur im Umlauf der Sterilisationsschritt durchgeführt wird, daß anschließend die medizinische Baugruppe mit Sterilwasser freigespült und anschließend unter Sterilbedingungen verschlossen wird.

Entsprechende Kombinationsmöglichkeiten bestehen bei der Vorrichtung.

Anhand von in den Zeichnungen dargestellten Ausführungsbeispielen mit Dialysatoren als medizinischen Baugruppen wird die Erfindung näher erläutert. Dabei werden ausgestaltende Merkmale der Erfindung sowie weitere Ausführungsformen deutlich.

Es zeigen:
- Figur 1:: Eine Sterilisationsanlage für Dialysatoren mittels Druckwasser als Sterilisiermedium.
- Figur 2:: Das Schaltpult einer Programmsteuerung zur Steuerung der Anlage nach Figur 1.
- Figur 3:: Einen Ausschnitt aus der Anlage nach Figur 1 mit alternativ einstellbaren Tanks für unterschiedliche Sterilisiermedien.
- Figur 3a:: Einen Ausschnitt aus der Anlage nach Figur 1 mit einem Behälter für Sterilluft zur Trocknung der Dialysatoren.

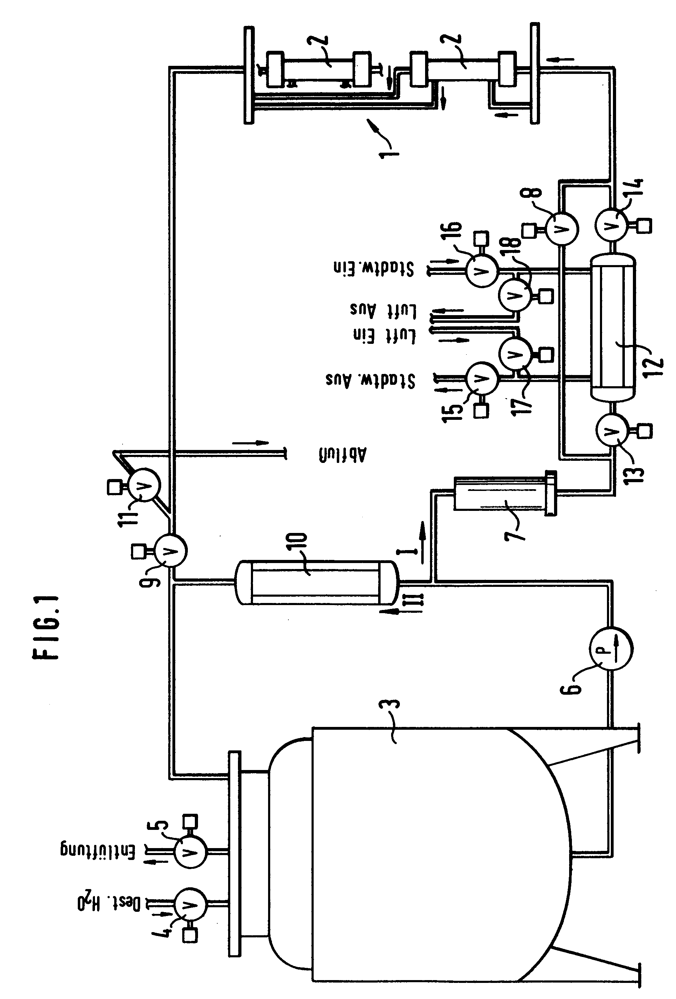

Die in Figur 1 dargestellte Sterilisationsanlage dient zum Sterilisieren von regenerierten Cellophan-Dialysatoren mittels Druckwasser bei einer Temperatur von vorzugsweise 121°C.

Die zu sterilisierenden Dialysatoren 2 - vorzugsweise 10 Stück - die sich jeweils auf einem geschliffenen Edelstahlblech befinden, werden jeweils in einer Spannstation 1 eingesetzt und mit ihren offenen Verschlüssen - blut- wie dialysatseitig - fest mit den Spülanschlüssen der Spannstation verbunden. Vorzugsweise wird eine Anschlußtechnik nach Patentanmeldung P 38 25 573 verwendet.

In einem Behälter 3 wird destiliertes Wasser mit einer Temperatur von 123°C bereit gehalten. Der Behälter weist ein Ventil 4 für die Einspeisung von destillertem Wasser sowie ein Ventil 5 für die Entlüftung des Behälters auf. Der Behälter steht unter erhöhtem Druck, damit das Wasser bei der vorgegebenen Temperatur nicht siedet.

Mittels einer Umwälzpumpe 6 wird das heiße Druckwasser dem Behälter entzogen. Ein Teilstrom I wird durch ein Filterelement 7 mit einer Filterporengröße von 10 mikrometer und ein erstes Kreislaufventil 8 durch die zu sterilisierenden Dialysatoren 2 und ein weiteres Kreislaufventil 9 zurück in den Behälter 1 gefördert. Ein anderer Teilstrom II wird ständig durch einen Wärmetauscher 10 in den Behälter zurück gefördert und dient zur Konstanthaltung der Behältertemperatur.

Stromab der Dialysator-Spannstation ist ein weiteres Ventil 11 vorgesehen, welches mit einem Abfluß verbunden ist.

Zwischen dem Filterelement 7 und der Spannstation 1 für die Dialysatoren ist ein Kühler 12 mit einem vor- und nachgeschalteten Ventil 13, 14 im Bypaß zum Kreislauf geschaltet. Auf der Kühlseite ist der Kühler mit Ventilen 15, 16 zum Ein- und Auslaß von Kühlwasser (Stadt- bzw. Leitungswasser) sowie mit Ventilen 17, 18 für eine Be- und Entlüftung versehen.

Das Sterilisieren läuft in mehreren Verfahrensschritten ab.

### 1. Einsetzen der Dialysatoren:

Alle Verschlußkappen der Dialysatoren 2 werden so aufgedreht, daß eine vollständige Verrastung und Abdichtung gewährleistet ist. Die Verschlußkappen - je Dialysator zwei Kappen für die Blutseite und zwei Kappen für die Dialysatseite - weisen einen O-Ring und einen eingesetzten Verschlußstopfen auf (nach DE 38 25 573 A 1).

Danach werden die Dialysatoren in die Aufnahmevorrichtung der Spannstation 1 eingesetzt, die Abdichtzylinder für die Dialysatorverschlüsse besitzen.

### 2. Spülen:

Nach dem Einsetzen der Dialysatoren 2 in die Spannstation werden die Ventile 5, 11, 13, 14 geöffnet; die anderen Ventile sind geschlossen. Die Dialysatoren 2 und der Kühler 12 werden mit heißem Druckwasser von ca. 98°C für ein bestimmtes Zeitinterwall (ca. 4 Minuten) durchgespült, wobei das Spülwasser direkt in den Abfluß gelangt und somit dem Gesamtkreislauf entzogen wird. Während der Spülphase steigt die Temperatur durch die Aufheizung mittels des Wärmetauschers 10 im Tankbypaß II ständig an.

### 3. Sterilisieren:

Nach Beendigung des Spülvorganges erfolgt - bei weiterhin geöffneten Ventilen 13 und 14 unter gleichzeitigem Öffnen der Ventile 8 und 9 - das Aufheizen des Druckwassers auf die Sterilisationstemperatur von ca. 123°C. Die Aufheizphase ist so bemessen, daß nach ihrem Ablauf - ca. 10 Minuten - an allen Meßstellen mindestens eine Temperatur von 121°C angezeigt wird. Das heiße Druckwasser wird mittels der Umwälzpumpe 6 ständig im Kreislauf über das Filterelement 7 und die Dialysatoren 2 zurück in den Behälter 1 geführt. Der Sterilisationsvorgang einschließlich des Aufheizens beträgt ca. 30 Minuten. Es muß dabei sichergestellt sein, daß an allen Meßstellen während der Gesamtdauer der Sterilisationsphase mindestens eine Temperatur von 121°C herrscht.

### 4. Kühlen:

Nach dem Sterilisieren wird das Druckwasser durch Einschaltung des - vorher sterilisierten-Kühlers 12 mittels der Ventile 15, 16, unter gleichzeitigem Schließen des Kreislaufventiles 8, auf Raumtemperatur abgekühlt und in gekühlter Form durch die Dialysatoren gefördert, die dadurch gleichfalls auf Raumtemperatur abgekühlt werden. Die Nachkühlung im Blutteil des Dialysators ist dabei etwas niedriger als im Dialysatteil (25° gegenüber 20°).

### 5. Verschließen und Entnehmen:

Nach dem Abkühlen wird der Dialysator durch pneumatisches Einschlagen der Verschlußstopfen in die Verschlußkappen automatisch verschlossen und aus der Spannstation 1 durch Entspannen und Zurückfahren der Haltezylinder entnommen. Das abgekühlte sterile Wasser verbleibt im sterilen Dialysator.

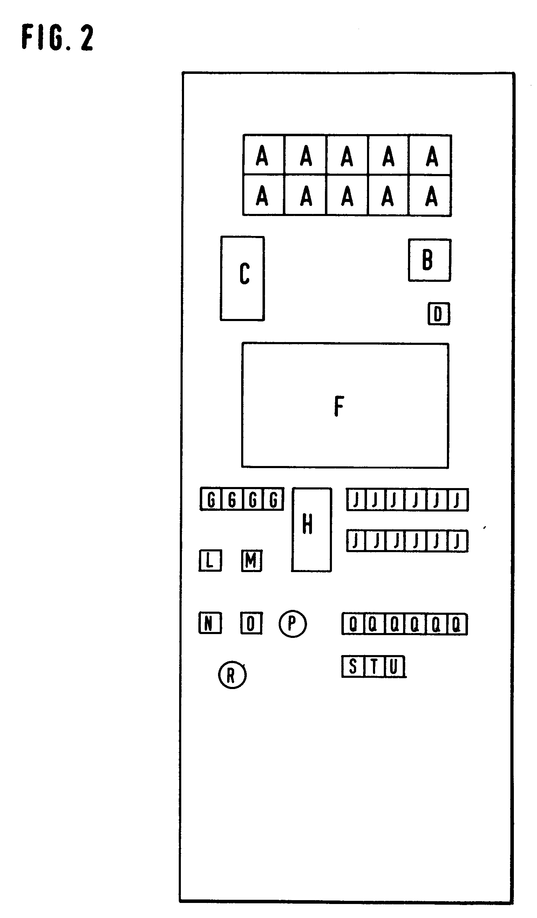

Die Steuerung der Sterilisationsanlage kann von Hand oder - alternativ durch einen Betriebsartenschalter einschaltbar - im Automatikbetrieb erfolgen. Im Handbetrieb sind die Ventile einzeln ansteuerbar. Im Automatikbetrieb sorgt eine Programmsteuerung für das zeit- und temperaturgerechte Steuern der Ventile. Derartige Programmsteuerungen, die nacheinander zeit- und sensorgesteuert einzelne Verfahrensschritte im Ablauf steuern, sind dem einschlägigen Fachmann bekannt. Die zugehörige Steuertafel weist zweckmäßig eine Gestaltung nach Figur 2 auf und enthält folgende Anzeige- und Steuerelemente, die jeweils in der Figur 2 mit einem Buchstaben gekennzeichnet sind, und die jeweils mit den zugehörigen Sensoren bzw. Steuerteilen verbunden sind.
A Digitalanzeigen für Temperatur
B Digitalanzeige Behältertemperatur
C Füllstandsanzeige
D Umschalter Temperaturanzeige für Blut und Dialysat
F Schreiber für Druck und Temperatur
G Kontrollampen für Pumpen
H Regler für Beheizungstemperatur
I Umschalter Hand-Null-Automatik für pneumatische Ventile
L Schalter für Reservepumpe
M Schalter für Pumpe
N Taster "Start"
O Taster "Stop"
P "Not Aus"
Q Programm-Meldeleuchten
R Hauptschalter
S Taster "Spannen"
T Taster "Verschließen"
U Taster "Entnehmen"

Die Bedienung und Einstellung der Sterilisationsanlage im Automatikbetrieb läuft daher wie folgt ab:

Die Dialysefilter, beispielsweise zehn Stück, werden in zehn Spannstationen eingesetzt und durch Betätigen des Tasters S mit der Bezeichnung "Spannen" an die Anlage angeschlossen. Durch Betätigen des Tasters N "Start" wird der automatische Ablauf in Bewegung gesetzt. Die einzelnen Programmschritte - Spülen, Sterilisieren, Kühlen, Verschließen und Entnehmen - laufen, je nach eingestellter Zeit, selbsttätig programmgesteuert nacheinander ab.

Während des Programmablaufes werden durch Meldeleuchten Q die einzelen Programmschritte angezeigt. An den Digitalanzeigen A werden die Temperaturen von jedem Dialysator - mittels des Umschalters D wechselweise blut- und dialysatseitig - gezeigt und auf dem Schreiber F zur Dokumentation mitgeschrieben. Beim Aufleuchten der Lampe "Ende" ist das Programm abgelaufen. Die Dialysatoren können nun, nachdem die Tasten T "Verschließen" und U "Entnehmen" betätigt wurden, aus den Spannstationen entnommen werden.

Nachdem wieder neue Dialysatoren in die Spannstationen eingesetzt sind, kann der Vorgang erneut gestartet werden.

Im Ausführungsbeispiel nach Figur 1 mit Cuprophan-Dialysatoren wird heißes, destilliertes Druckwasser von mindestens 121°C als Sterilisiermedien verwendet. Anstelle des Druckwassers kann auch eine 3 %ige Peressigsäure bei Raumtemperatur als Sterilisiermedium verwendet werden, wobei dann die Dialysatoren nach der Sterilisierung mit Sterilwasser von dem Sterilisiermedium freigespült werden müssen.

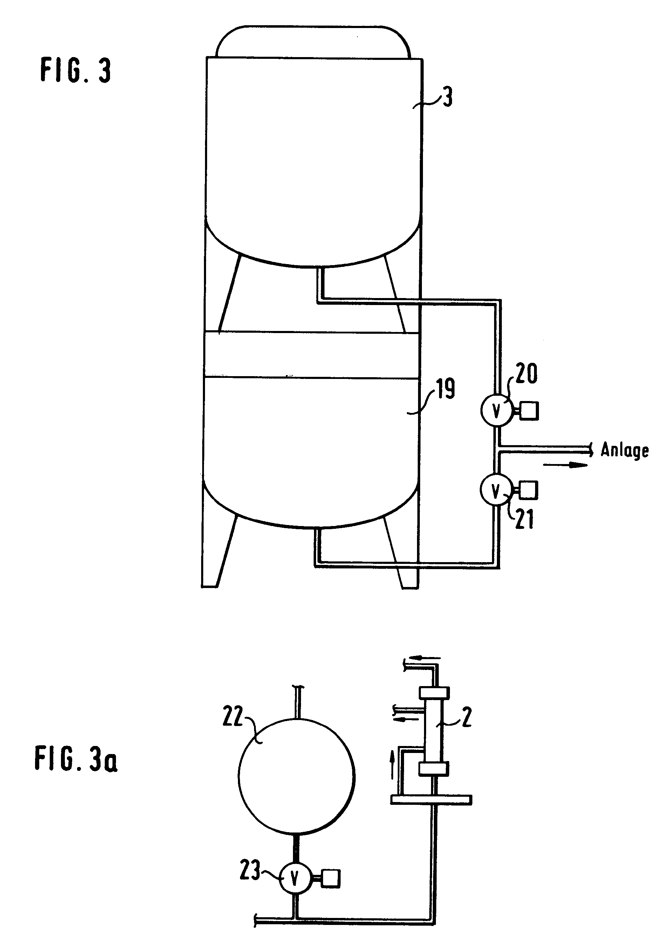

Anlagentechnisch ist es dabei - wie in Figur 3 ausschnittweise dargestellt - zweckmäßig, Behälter 3, 19 für beide Sterilisiermedien vorzusehen, die - über zugeordnete Ventile 20, 21 - alternativ zuschaltbar sind. Der Tank 3 mit dem heißen Wasser kann dabei als Quelle für das Sterilwasser zum Freispülen des Dialysators nach seiner Sterilisation mit Peressigsäure aus dem Behälter 19 dienen. Nach dem Freispülen mit Sterilwasser können die Dialysatoren 2 durch Einspeisung von Sterilluft aus einem Behälter 22, zuschaltbar über das Ventil 23 gemäß Figur 3a in das System, getrocknet werden, oder das Sterilwasser verbleibt entsprechend dem Ausführungsbeispiel nach Figur 1 im Dialysator.

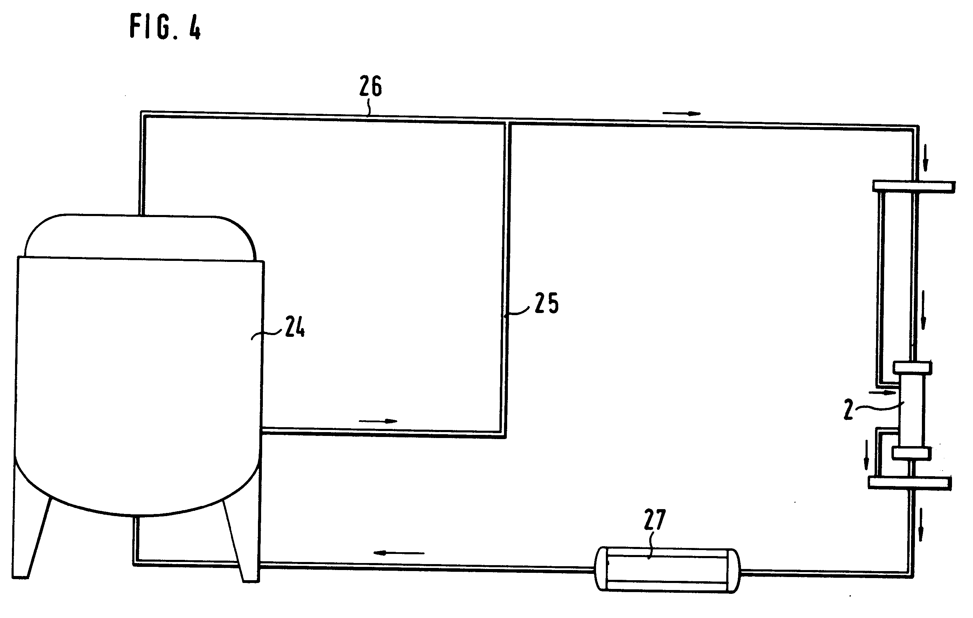

Als Sterilisiermedium ist ferner Wasserdampf verwendbar, der dann, anlagentechnisch gemäß der schematischen Darstellung ohne Ventile nach Figur 4, aus einer Dampfquelle 24 bereitgestellt wird. Dieses Sterilisiermedium wird bevorzugt bei Dialysatoren 2 mit Polysulfon-Membranen verwendet. Das Polysulfon ist bekanntermaßen hydrophob und enthält als Hydrophilierungsmittel PVP (Polyvinyl-pyrrolidon).

Analog dem dargestellten Ausführungsbeispiel in Figur 1 erfolgt zunächst ein Spülen des Dialysators 2 mit Wasser aus einer entsprechenden Quelle, z.B. dem Kondensat der Dampfquelle 24 über die Leitung 25. Danach erfolgt die Heißsterilisation mit Wasserdampf über die Leitung 26, der anschließend einem Kondensator 27 zugeführt wird. Nach der Sterilisation wird der Dialysator 2 mit Sterilwasser, z.B. aus dem Kondensat der Dampfquelle 24, nachgespült, um das herausgelöste PVP zu entfernen. Anschließend wird der Dialysator 2 mit Sterilluft getrocknet, die durch eine geeignete Quelle bereitgestellt und in die Anlage eingespeist wird, z.B. gemäß Figur 3a.

Die anlagentechnische Realisierung vorgenannter Schritte und das Vorsehen der notwendigen Steuerung ist dem Fachmann aufgrund der vorgegebenen Verfahrensschritte und in Anlehnung an das beschriebene Ausführungsbeispiel nach Figur 1 ohne erfinderisches Bemühen möglich. Beispielsweise entfällt bei der Behandlung mit Peressigsäure nach Figur 3 als Sterilisiermedium mit Raumtemperatur die Kühlphase. In einem derartigen Fall ist entweder in der Anlage kein entsprechender Kühler 12 vorhanden oder er bleibt ausgeschaltet.

## Patentansprüche

1. Verfahren zur Sterilisation einer medizinischen Baugruppe mit mindestens zwei Anschlüssen zur Einschaltung in einen Flüssigkeitsstrom eines medizinischen Gerätes, bei dem die Baugruppe von einem Sterilisiermedium durchströmt wird,
gekennzeichnet durch folgende Verfahrensschritte:
a. Einkoppeln der Baugruppe in ein Sterilisier-System unter druckdichtem Ankoppeln der beiden Anschlüsse an eine Zu- und Ableitung des Systems unter atmosphärischen Bedingungen,
b. Durchströmen der Baugruppe mit dem Sterilisiermedium des Systems für einen vorgegebenen Zeitraum,
c. Keimdichtes Verschließen der beiden Anschlüsse der Baugruppe im angekoppelten Zustand, ohne diese zu öffnen, nach Abschluß der Sterilisation,
d. Entnahme der Baugruppe aus dem Sterilisier-System als sterile Einheit mit verschlossenen Anschlüssen.

2. Verfahren nach Anspruch 1, dadurch gekennzeichnet, daß das Sterilisiermedium Druckwasser von mindestens 105°C oder Peressigsäurelösung ist.

3. Verfahren nach Anspruch 1 oder 2, dadurch gekennzeichnet, daß vor der Beaufschlagung der Baugruppe mit dem Sterilisiermedium diese mit einer Flüssigkeit aus dem System, insbesondere Wasser, durchspült wird.

4. Verfahren nach Anspruch 1, dadurch gekennzeichnet, daß nach Abschluß der Sterilisation vor dem Verschließen der Anschlüsse eine Spülung der Baugruppe mit einer sterilen Flüssigkeit, insbesondere Wasser oder dem abgekühlten Sterilisiermedium, durchgeführt wird.

5. Verfahren nach Anspruch 4, dadurch gekennzeichnet, daß zumindest ein Teil der sterilen Flüssigkeit beim Verschließen der Anschlüsse in der Baugruppe verbleibt.

6. Verfahren nach einem der Ansprüche 1 bis 4, dadurch gekennzeichnet, daß vor dem Verschließen der Anschlüsse der Baugruppe eine Trocknung mit einem sterilen Gas, vorzugsweise Luft von mindestens 50°C, insbesondere mindestens 100°C, durchgeführt wird.

7. Vorrichtung zur Sterilisation einer medizinischen Baugruppe mit mindestens zwei Anschlüssen zur Einschaltung in einen Flüssigkeitsstrom eines medizinischen Gerätes, bei dem die Baugruppe von einem Sterilisiermedium durchströmt wird, mit einer Einrichtung zur Erzeugung und/oder Bereitstellung des Sterilisiermediums im System, gekennzeichnet durch
- mindestens eine Atmosphärendruck ausgesetzte Spannstation (1) für die temporäre druckdichte Ankopplung der Baugruppen-Anschlüsse, welche Spannstation einen Zulauf und einen Abfluß aufweist,
- mindestens eine Rohrleitung von der Sterilisiermedium-Einrichtung (3, 19, 24) zum Zulauf der Spannstation,
- mindestens eine Rohrleitung, die am Abfluß der Spannstation (1) angeschlossen ist,
- Verschlußorgane (8, 9, 11, 13 - 18), insbesondere Absperrventile, in den Rohrleitungen,
- zumindest eine Einrichtung zum keimdichten Verschließen der Anschlüsse der Baugruppe (2) im angekoppelten Zustand.

8. Vorrichtung nach Anspruch 7, dadurch gekennzeichnet, daß die Rohrleitungen zu einem geschlossenen, einen Kreislauf vorgebenden System zusammengeschaltet sind, in das zumindest ein Tank (3, 19, 24) zur Aufnahme des Sterilisiermediums und vorzugsweise ein Wärmetauscher (10) geschaltet ist.

9. Vorrichtung nach Anspruch 7 oder 8, dadurch gekennzeichnet, daß in der zum Zulauf der Spannstation (1) führenden Rohrleitung eine Abzweigung vorgesehen ist, die mit einer Anordnung (22, 23) zur Erzeugung und/oder Bereitstellung eines zweiten Mediums, vorzugsweise eines sterilen Fluids, insbesondere Sterilwasser oder Sterilgas (Sterilluft), in Verbindung steht (Fig. 3a).

10. Vorrichtung nach einem der Ansprüche 7 bis 9 , dadurch gekennzeichnet, daß für die Einleitung und Beendigung der einzelnen Verfahrensschritte eine selbsttätige Steuerung, insbesondere der Ventile, vorgesehen ist.

11. Vorrichtung nach Ansprüchen 7 bis 10, gekennzeichnet durch
- einen Druckbehälter (3) zur Aufnahme von destilliertem Wasser,
- ein Rohrleitungssystem, welches den Druckbehälter (3) mit der Spannstation (1) im Kreislauf verbindet,
- eine Umwälzpumpe (6), Absperrventile (8, 9) und einen mikroporösen Filter (7) stromauf der Spannstation (1) im Kreislauf,
- einen Kühler (12) im durch Ventile (13, 14) absperrbaren Bypass zu dem Kreislauf zwischen dem aufstromigen Ende der Spannstation (1) und dem mikroporösen Filter (7),
- eine mittels Ventil (11) verschließbare Abflußleitung am abstromigen Ende der Spannstation (1) und
- einen nach der Umwälzpumpe (6) im Bypass zum Druckbehälter (3) geschalteten Wärmetauscher (10) zur Behältertemperierung (Fig. 1), wobei vorzugsweise zusätzlich ein Behälter (19) mit Peressigsäure, der über Ventile (20, 21) alternativ zu dem Druckbehälter (3) in den Kreislauf einschaltbar ist (Fig. 3) oder ein Dampferzeuger (24) zur Bereitstellung des Sterilisiermediums vorgesehen ist (Fig. 4).

## Claims

1. A method for sterilizing a medical unit comprising at least two connections for insertion into a fluid flow of a medical device where a sterilizing medium flows through said unit,
characterized by the following steps:
a. inserting said unit into a sterilization system under pressure-tight coupling of both connections to a feed conduit and discharge conduit of the system under atmospheric conditions;
b. passing said sterilizing medium from said system through said unit for a given period;
c. germproof closing of both connections of said unit in the coupled state, without the same being opened, on completion of the sterilization operation;
d. removing said unit from said sterilization system as a sterile unit with closed connections.

2. A method according to claim 1, characterized in that said sterilizing medium is pressurized water of at least 105°C or peracetic acid solution.

3. A method according to claim 1 or 2, characterized in that prior to the treatment of said unit with said sterilizing medium, a fluid from said system, in particular water, is flushed through said unit.

4. A method according to claim 1, characterized in that on completion of the sterilization operation said unit is flushed with a sterile fluid, in particular water or said cooled sterilization medium, prior to the closing of said connections.

5. A method according to claim 4, characterized in that at least a part of said sterile fluid remains in said unit when said connections are closed.

6. A method according to any of claims 1 to 4, characterized in that prior to the closing of said connections of said unit a drying operation with a sterile gas,
a. preferably air of at least 50°C, in particular,
b. at least 100°C,
is carried out.

7. An apparatus of sterilizing a medical unit comprising at least two connections for insertion into a fluid flow of a medical device where a sterilizing medium flows through said unit, comprising a means for generating and/or providing said sterilizing medium in said system, characterized by
- at least one fastening means which is subjected to an atmospheric pressure and used for the temporary pressure-tight coupling of said unit connections and comprises an inlet and an outlet,
- at least one conduit from said sterilizing medium means (3, 19, 24) to the inlet of said fastening means,
- at least one conduit connected to the outlet of said fastening means (1),
- closing members (8, 9, 11, 13-18), in particular shut-off valves, in said conduits,
- at least one means for the germproof closing of said connections of said unit (2) in the coupled state.

8. An apparatus according to claim 7, characterized in that said conduits are combined to form a closed system defining a circuit, in which system at least one tank (3, 19, 24) for receiving said sterilizing medium is arranged and preferably a heat exchanger (10).

9. An apparatus according to claim 7 or 8, characterized in that said conduit leading to said inlet of said fastening means (1) has provided therein a branch which communicates with an assembly (22, 23) for generating and/or providing a second medium, preferably a sterile fluid, in particular sterile water or sterile gas (sterile air) (Fig. 3a).

10. An apparatus according to any of claims 7-9, characterized in that an automatic control is provided for starting and ending the individual process steps, in particular of the valves.

11. An apparatus according to claims 7-10, characterized by
- a pressure container (3) for receiving distilled water,
- a conduit system for connecting said pressure container (3) to said fastening means (1) in said circuit,
- a circulating pump (6), shut-off valves (8, 9) and a microporous filter (7) upstream of said fastening means (1) in said circuit,
- a cooler (12) in said bypass, which is adapted to be shut off by valves (13, 14), to the circuit between the upstream end of said fastening means (1) and said microporous filter (7),
- an outlet conduit which is adapted to be closed by means of a valve (11) and provided at the downstream end of said fastening means (1), and
- a heat exchanger arranged after said circulating pump (6) in said bypass to said pressure container (3) for controlling the temperature of said container (Fig. 1), with preferably an additional container (19) with peracetic acid which is alternatively connectable instead of said pressure container (3) via valves (20, 21) into said circuit (Fig. 3) or a steam generator (24) for generating said sterilizing medium (Fig. 4) being provided.

## Revendications

1. Procédé pour la stérilisation d'un sous-ensemble médical comportant au moins deux raccords en vue de son branchement dans un circuit de liquide d'un appareil médical, dans lequel le sous-ensemble est traversé par un milieu de stérilisation,
caractérisé par les étapes suivantes :
a. montage du sous-ensemble dans un système de stérilisation par raccordement étanche à la pression des deux raccords à une conduite d'alimentation et une conduite d'évacuation du système dans des conditions atmosphériques,
b. balayage du sous-ensemble au moyen du milieu de stérilisation du système pendant une période prédéterminée,
c. fermeture étanche aux bactéries des deux raccords du sous-ensemble à l'état raccordé, sans les ouvrir au terme de la stérilisation,
d. enlèvement du sous-ensemble du système de stérilisation en tant qu'unité stérile à raccords fermés.

2. Procédé suivant la revendication 1, caractérisé en ce que le milieu de stérilisation est de l'eau sous pression à au moins 105°C ou une solution d'acide peracétique.

3. Procédé suivant la revendication 1 ou 2, caractérisé en ce qu'avant d'alimenter le sous-ensemble au moyen du milieu de stérilisation, on le rince au moyen d'un liquide provenant du système, en particulier de l'eau.

4. Procédé suivant la revendication 1, caractérisé en ce qu'au terme de la stérilisation, avant la fermeture des raccords, un rinçage du sous-ensemble au moyen d'un liquide stérile, en particulier de l'eau, ou le milieu de stérilisation refroidi, est effectué.

5. Procédé suivant la revendication 4, caractérisé en ce qu'au moins une fraction du liquide stérile reste dans le sous-ensemble lors de la fermeture des raccords.

6. Procédé suivant l'une quelconque des revendications 1 à 4, caractérisé en ce qu'avant la fermeture des raccords du sous-ensemble, un séchage au moyen d'un gaz stérile, de préférence de l'air à au moins 50°C, en particulier au moins 100°C, est effectué.

7. Installation pour la stérilisation d'un sous-ensemble médical pourvu d'au moins deux raccords en vue de son branchement dans un circuit de liquide d'un appareil médical dans laquel le sous-ensemble est traversé par un milieu de stérilisation, comportant un dispositif pour produire et/ou préparer le milieu de stérilisation dans le système, caractérisé par
- au moins un poste de serrage (1) soumis à la pression atmosphérique, pour le raccordement temporaire étanche à la pression des raccords des sous-ensembles, ce poste de serrage présentant une admission et une évacuation,
- au moins une conduite allant du dispositif à milieu de stérilisation (3, 19, 24) à l'admission du poste de serrage,
- au moins une conduite qui est raccordée à l'évacuation du poste de serrage (1),
- des organes de fermeture (8, 9, 11, 13 - 18), en particulier des valves d'arrêt dans les conduites,
- au moins un dispositif pour fermer, d'une manière étanche aux bactéries, les raccords du sous-ensemble (2) dans l'état raccordé.

8. Installation suivant la revendication 7, caractérisée en ce que les conduites sont raccordées en un système clos prédéfinissant un circuit dans lequel sont branchés au moins un réservoir (3, 19, 24) destiné à recevoir le milieu de stérilisation et de préférence un échangeur de chaleur (10).

9. Installation suivant la revendication 7 ou 8, caractérisée en ce que, dans la conduite aboutissant à l'admission du poste de serrage (1) est prévu un branchement qui est en communication avec un dispositif (22, 23) pour produire et/ou préparer un second milieu, de préférence un fluide stérile, en particulier de l'eau stérile ou du gaz stérile (air stérile) (Fig. 3a).

10. Installation suivant l'une quelconque des revendications 7 à 9, caractérisée en ce qu'une commande automatique, en particulier des valves, est prévue pour l'amorçage et la terminaison des étapes individuelles du procédé.

11. Installation suivant les revendications 7 à 10, caractérisée par
- un récipient à pression (3) destiné à recevoir de l'eau distillée,
- un système de conduites raccordant en circuit le récipient à pression (3) au poste de serrage (1),
- une pompe de circulation (6), des valves d'arrêt (8, 9) et un filtre microporeux (7) en amont du poste de serrage dans le circuit,
- un refroidisseur (12) dans la dérivation du circuit pouvant être fermée par les valves (13, 14), entre l'extrémité d'amont du poste de serrage (1) et le filtre microporeux (7),
- une conduite d'évacuation pouvant être fermée au moyen d'une valve (11) à l'extrémité d'aval du poste de serrage (1), et
- un échangeur de chaleur (10) branché en aval de la pompe de circulation (6) dans la dérivation du récipient à pression (3) pour l'équilibrage de la température du récipient (Fig. 1), étant entendu qu'en outre, est prévu un récipient (19) contenant de l'acide peracétique qui peut être branché via des valves (20, 21) dans le circuit (Fig. 3) à titre d'alternative au récipient à pression (3) ou un générateur de vapeur (24) pour la préparation du milieu de stérilisation (Fig. 4).
